Europäisches Patentamt

European Patent Office

Office européen des brevets

(19)

(11) Publication number: **0 106 940**
**B1**

(12) EUROPEAN PATENT SPECIFICATION

(45) Date of publication of patent specification: **25.02.87**

(21) Application number: **83106547.9**

(22) Date of filing: **05.07.83**

(51) Int. Cl.⁴: **G 01 F 1/58** // A61M1/14

(54) **An arrangement for the measurement of the difference between two flows in two separate ducts.**

(30) Priority: **28.09.82 SE 8205529**

(43) Date of publication of application:
**02.05.84 Bulletin 84/18**

(45) Publication of the grant of the patent:
**25.02.87 Bulletin 87/09**

(84) Designated Contracting States:
**AT BE CH DE FR GB IT LI LU NL**

(56) References cited:
**EP-A-0 052 004**
**DE-A-2 214 735**
**GB-A-2 003 274**
**GB-A-2 056 691**

(73) Proprietor: **Gambro Lundia AB**
**Box 10101**
**S-220 10 Lund (SE)**

(72) Inventor: **Gummesson, Bengt-Ake Göran**
**Box 48**
**S-230 40 Bara (SE)**
Inventor: **Holmberg, Bengt Magnus**
**Svalvägen 27**
**S-230 50 Bjärred (SE)**
Inventor: **Jönsson, Sven Anders**
**Rydbergs väg 12**
**S-245 00 Staffanstorp (SE)**
Inventor: **Mattisson, Ulf Kenneth**
**Ollonborrevägen 11**
**S-240 17 S. Sandby (SE)**

(74) Representative: **Boberg, Nils Gunnar Erik**
**Gambro AB Patent Department Box 10101**
**S-220 10 Lund (SE)**

Courier Press, Leamington Spa, England.

## Description

### Technical fields

The present invention relates to an arrangement for the measurement of the difference between the rates of two mass flows in two separate ducts with one or more monitoring electrodes, arranged in each duct.

The invention relates first and foremost to the measurement of relatively small differences between two relatively large mass flows. The invention may be applied, for example, to the measurement of ultrafiltration in a dialysis system, where the continuing clean dialysis solution flows through the one duct, whilst the same solution, but increased through ultrafiltration in the dialyzer, flows through the other duct. The two main mass flows may be of the order of magnitude 500 ml/min whereas the difference, that is to say the ultrafiltration, may be somewhere between 0 and 40 ml/min and exceptionally a little higher.

### Background art

In British patent applications GB—A—2 003 274 and GB—A—2 056 691 two similar systems of electromagnetic measurement are described. In both systems the mass flows whose difference is to be measured pass through two parallel ducts. In accordance with the first-mentioned publication the measurement is performed with the help of three electrodes which are acted upon as a function of the flowing medium and an external magnetic field. In accordance with the last-mentioned publication two main electrodes which cooperate with a number of earth electrodes are instead acted upon in the same manner.

Both systems suffer from the disadvantage that the flowing media act upon the two ducts, or the electrodes, in a different manner. If, for example, the designs are used for the measurement of ultrafiltration in dialysis, deposits from the contaminated liquid are obtained on the electrodes and/or on the actual duct walls. Even a deposit in the order of magnitudes of a few μm may cause substantial errors in the readings.

### Disclosure of invention

The invention thus relates to an arrangement for the measurement of the difference between the rates of two mass flows in two separate ducts with one or more monitoring electrodes arranged in each duct. The above-mentioned problem is solved in that valve means are provided for the alternate change-over of the mass flows from one duct to the other duct and vice-versa, so that ultimately all electrodes and/or duct walls are acted upon to a substantially equal extent. This realization has the advantage that the dialysis or any other continuing treatment can proceed without interruption.

Alternatively valve means are provided for the conducting of one and the same mass flow through both ducts, providing a zero difference value to be used as a new zero value for the continued measurement.

If the lastmentioned system is used for the measurement and possible control of the mass flow to and from a dialyzer, the same is appropriately provided with means for the conducting of the clean dialysis solution through both the ducts. At the same time means can be provided for the by-passing of the mass flow from the dialyzer past the two ducts thus allowing the dialysis to be continued, at least to a limited extent, during the zeroing or calibration. At the same time a certain clean-flushing of the contaminated duct is achieved.

The invention is used preferably for an arrangement wherein the said electrodes are arranged in a manner known in itself in a magnetic field crossing the two ducts. Signals emitted from the electrodes can then be picked up and used directly for the adjustment in case of large differences in the mass flows, whilst the difference in signals can be picked up and used for adjustment in case of small differences in the mass flows.

### Brief description of drawings

Fig. 1 shows schematically a block diagram of an arrangement in accordance with the invention in a preferred embodiment.

Fig. 2 and 3 likewise schematically show alternative possibilities of connection for two parallel mass flows whose differences in flow rates can be measured with the help of the arrangement in accordance with the invention.

### Best mode of carrying out the invention

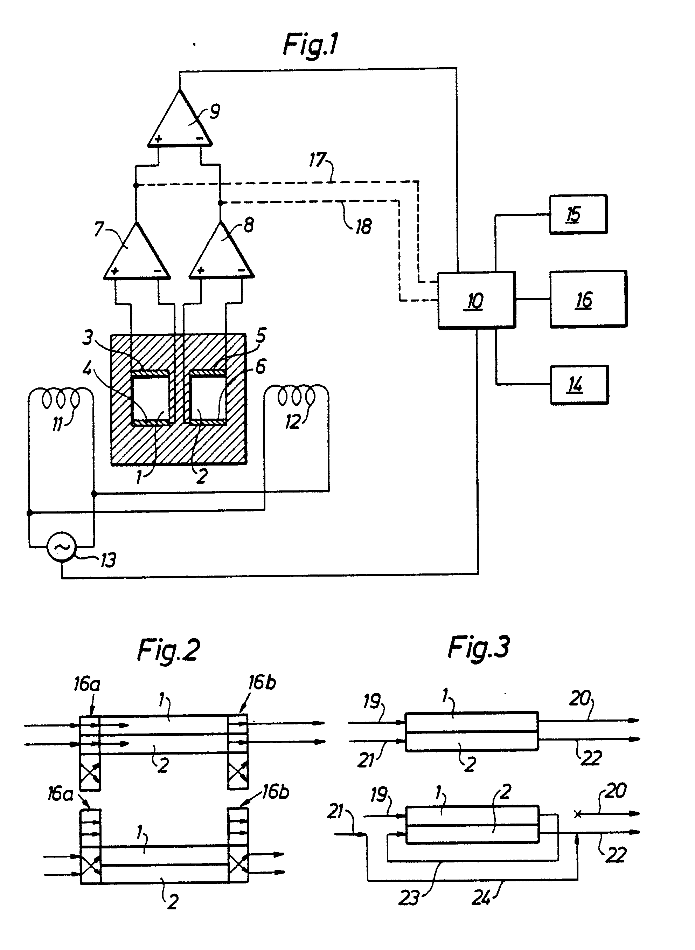

In Fig. 1 are shown two ducts 1 and 2 with four electrodes 3, 4, 5 and 6 which are connected to differential measuring instruments ·7 and 8 respectively which in turn are connected to a differential measuring instrument 9. All differential measuring instruments are connected to a microcomputer 10. The mass flows in the two ducts 1 and 2 are subjected to a magnetic field generated with the help of the coils 11 and 12, operated by a source of current 13, which in turn is controlled by the computer 10. It is shown schematically how the computer can be provided with a display 14 and a controller 15. At the same time the microcomputer can be adapted so as to control one or more valves 16. With regards to the theories behind the measurement in accordance with the system shown, reference is made to the aforementioned British publications. In this connection it may be mentioned that the broken lines 17 and 18 are intended to indicate that the measured values obtained from the differential measuring instruments 7 and 8 are used directly for the control of the computer 10. This is done in the case of large differences between the flows in the ducts 1 and 2. In the case of small differences the difference obtained from the differential measuring instrument 9 is used instead for the control of the microcomputer 10. Naturally the system may be provided for this purpose with different types of amplifiers, rectifiers, earth electrodes and other electric components which are normal for anyone versed in the art.

In Fig. 1 the ducts 1 and 2 are shown in a cross-section transversely to the direction of flow. In Fig. 2 the corresponding ducts are shown instead in their longitudinal direction. Ducts 1 and 2 are combined with two valve blocks 16a and 16b by means of which the mass flows through ducts 1 and 2, respectively, can be changed over alternately to the opposite duct, so that ultimately all electrodes and/or duct walls are acted upon to a substantially equal extent.

In Fig. 3 the ducts 1 and 2 are also shown in longitudinal direction. If the system in accordance with Fig. 3 is used, for example, in dialysis, the arrow 19 may designate the mass flow from a control unit normally used for dialysis. The arrow 20 in this case designates the mass flow to the actual dialyzer and the arrow 21 the mass flow from the dialyzer. The arrow 22 in this case designates the mass flow to a drain and/or to a possible regeneration and recirculation. In the bottom part of Fig. 3 the arrow 23 indicates how the clean dialysis solution can be conducted first through the duct 1 and then through the duct 2 to be removed finally to a drain or the like without passing the dialyzer. At the same time the arrow 24 is meant to indicate how the mass flow from the dialyzer can be conducted, past the two ducts 1 and 2, directly to the drain. The system in accordance with Fig. 3 has the advantage over the system in accordance with Fig. 2 that there is no risk of any particles broken loose from a previously contaminated duct being passed to the dialyzer. In accordance with this system any contaminations broken off are passed instead directly to the drain.

Naturally the invention is not limited simply to the preferred embodiment described above, but can be varied within the scope of the following claims. The invention is also applicable, for example, to systems with more or fewer electrodes. Likewise it is not necessary to have parallel flow, but the system can also be adapted to counter-current flow. Finally, it may be mentioned that the calibration in accordance with the system shown in Fig. 3 can be repeated more or less frequently at regular intervals. In the case of dialysis it has been found to be appropriate to calibrate once every half hour. The calibration may proceed in such a manner that the mass flow difference is measured 300—400 times in a minute, whereupon a mean value is calculated which is substantially independent of any background noise.

## Claims

1. An arrangement for the measurement of the difference between the rates of two mass flows in two separate ducts (1, 2) with one or more monitoring electrodes (3—6) arranged in each duct, characterized by valve means (16; 16a; 16b) for the alternate change-over of the mass flows from one duct to the other duct and vice versa.

2. An arrangement for the measurement of the difference between the rates of two mass flows in two separate ducts (1, 2) with one or more monitoring electrodes (3—6) arranged in each duct, characterized by valve means (23) for conducting one and the same mass flow through both ducts (1, 2), providing a zero difference value to be used as a new zero value for the continued measurement.

3. An arrangement in accordance with claim 2, intended for the measurement and possible control of the mass flow to and from a dialyzer, characterized by valve means (23) for the conducting of the clean dialysis solution through both the ducts (1, 2).

4. An arrangement in accordance with claim 3, characterized by valve means (24) for the by-passing of the flow from the dialyzer past the two ducts (1, 2).

5. An arrangement in accordance with any one of the preceding claims, characterized in that the said electrodes (3—6) are arranged in a manner known in itself in a magnetic field crossing the two ducts (1, 2), making it possible for outgoing signals to be picked up and used directly for adjustment in case of large differences in the mass flows, whilst the difference in signals is picked up and used for adjustment in case of small differences in the mass flows.

## Patentansprüche

1. Anordnen zum Messen der Differenz zwischen den Geschwindigkeiten zweier Massenströme in zwei getrennten Leitungen (1, 2) mit einer oder mehreren Überwachungselektroden (3—6), die in jeder Leitung angeordnet sind, gekennzeichnet durch Ventileinrichtungen (16; 16a; 16b) für das abwechselnde Umschalten der Massenströme von einer Leitung zur anderen Leitung und umgekehrt.

2. Anordnung für das Messen der Differenz zwischen den Geschwindigkeiten zweier Massenströme in zwei getrennten Leitungen (1, 2) mit einer oder mehreren Überwachungselektroden (3—6), die in jeder Leitung angeordnet ist oder sind, gekennzeichnet durch Ventileinrichtungen (23) zum Führen ein und desselben Massenstromes durch beide Leitungen (1, 2), wobei ein Nulldifferenzwert vorgesehen ist, der als neuer Nullwert für die fortgesetzte Messung zu benutzen ist.

3. Anordnung nach Anspruch 2 für die Messung und mögliche Steuerung des Massenflusses zu einem Dialysator und von einem solchen her, gekennzeichnet durch Ventileinrichtungen (23) für das Führen der sauberen Dialyselösung durch beide Leitungen (1, 2).

4. Anordnung nach Anspruch 3, gekennzeichnet durch Ventileinrichtungen (24) für die By-pass-Führung des Stromes aus dem Dialysator an den zwei Leitungen (1, 2) vorbei.

5. Anordnung nach einem der vorhergehenden Ansprüche, dadurch gekennzeichnet, daß die Elektroden (3—6) in einer an sich bekannten Weise in einem Magnetfeld angeordnet sind, welches die zwei Leitungen (1, 2) kreuzt, wobei die Aufnahme herausgehender Signale ermöglicht wird, welche direkt für die Einstellung im Falle großer Differenzen in den Massenströmen benutzt werden, während die Differenz in den Signalen aufgenommen und für die Einstellung im Fall kleiner Differenzen in den Massenströmungen benutzt wird.

**Revendications**

1. Dispositif de mesure de la différence entre les débits de deux écoulements dans deux conduites séparées (1, 2) au moyen d'une ou plusieurs électrodes de mesure (3—6) prévues dans chaque conduite, caractérisé en ce qu'il comprend des moyens d'obturation (16; 16a; 16b) pour la permutation alternée des écoulements d'une conduite à l'autre conduite et vice versa.

2. Dispositif de mesure de la différence entre les débits de deux écoulements dans deux conduites séparées (1, 2) au moyen d'une ou plusieurs électrodes de mesure (3—6) prévues dans chaque conduite, caractérisé en ce qu'il comporte des moyens d'obturation (23) pour diriger un seul et même écoulement dans les deux conduites (1, 2), de manière à fournir une valeur de différence nulle à utiliser comme nouvelle valeur de zéro pour la suite de la mesure.

3. Dispositif suivant la revendication 2, destiné à la mesure et à la commande éventuelle de l'écoulement à destination et en provenance d'un dialyseur, caractérisé en ce qu'il comprend des moyens d'obturation (23) pour diriger la solution de dialyse propre à travers les deux conduites (1, 2).

4. Dispositif suivant la revendication 3, caractérisé en ce qu'il comprend des moyens d'obturation (24) pour le contournement de l'écoulement venant du dialyseur, au-delà des deux conduites (1, 2).

5. Dispositif suivant l'une quelconque des revendications précédentes, caractérisé en ce que lesdites électrodes (3—6) sont agencées d'une manière connue en elle-même dans un champ magnétique coupant les deux conduites (1, 2), ce qui permet de capter les signaux se sortie et de les utiliser directement pour le réglage dans le cas de grandes différences des écoulements, tandis que la différence entre les signaux est captée et utilisée pour le réglage dans le cas de petites différences des écoulements.

# Fig.1

# Fig.2

# Fig.3